# EUROPEAN PATENT SPECIFICATION

(11) **EP 2 747 822 B1**
(45) Date of publication and mention of the grant of the patent: **14.12.2016**
(21) Application number: 12791558.5
(22) Date of filing: 15.10.2012
(51) Int. Cl.: A61M 16/06, A62B 18/08

(54) **MULTIPLE-MATERIAL, SINGLE-PLANE HEADGEAR**
EINFLÄCHIGE KOPFBEDECKUNG AUS MEHREREN MATERIALIEN
FORCE EXTRA-ORALE À UN SEUL PLAN, À MULTIPLES MATÉRIAUX

(30) Priority: 03.11.2011 US 201161555104 P
(43) Date of publication of application: 02.07.2014
(73) Proprietor: Koninklijke Philips N.V., 5656 AE Eindhoven (NL)
(72) Inventor: CHODKOWSKI, Lauren Patricia, 5656 AE Eindhoven (NL); HO, Peter Chi Fai, 5656 AE Eindhoven (NL); BAIKO, Robert William, 5656 AE Eindhoven (NL)
(74) Representative: Steffen, Thomas
(86) International application number: PCT/IB2012/055597
(87) International publication number: WO 2013/064930

(56) References cited:
- WO-A1-92/01396
- GB-A- 1 389 095
- US-A- 3 026 522
- US-A- 3 337 381
- US-A- 4 719 136
- US-A- 5 341 609
- US-A1- 2002 007 896
- US-A1- 2008 047 560
- US-A1- 2011 253 143
- US-B1- 6 805 117
- JOHN M WASHBURN: 'Use of the Butted Seam by Textile Manufactures', [Online] 01 August 1931, pages 396 - 399, XP055229154 Retrieved from the Internet: <URL:http://www.cs.arizona.edu/patterns/wea ving/articles/wjm_butt.pdf> [retrieved on 2015-11-18]

## Description

### BACKGROUND OF THE INVENTION

### 1. Field of the Invention

The present invention pertains to materials for use in forming headgear, as well as headgear formed therefrom, for use in securing devices, such as respiratory masks, to a human head. The invention also pertains to methods of forming selected portions of a headgear.

### 2. Description of the Related Art

There are numerous situations where it is necessary or desirable to deliver a flow of breathing gas non-invasively to the airway of a patient, i.e., without intubating the patient or surgically inserting a tracheal tube in the patient's esophagus. For example, it is known to ventilate a patient using a technique known as non-invasive ventilation. It is also known to deliver continuous positive airway pressure (CPAP) or variable airway pressure, which varies with the patient's respiratory cycle, to treat a medical disorder, such as sleep apnea syndrome, in particular, obstructive sleep apnea (OSA), or congestive heart failure.

Non-invasive ventilation and pressure support therapies involve the placement of a respiratory patient interface device including a mask component that is typically secured on the face of a patient by a headgear assembly. The mask component may be, without limitation, a nasal mask that covers the patient's nose, a nasal cushion having nasal prongs that are received within the patient's nares, a nasal/oral mask that covers the nose and mouth, or a full face mask that covers the patient's face. It is known to maintain such devices on the face of a wearer by a headgear having one or more straps adapted to fit over/around the patient's head. Because such respiratory patient interface devices are typically worn for an extended period of time, it is important for the headgear to maintain the mask component in a desired position while doing so in a manner that is comfortable to the patient.

Conventional headgear assemblies are commonly formed of panels and/or straps formed from fabric that is die-cut from generally flat sheet materials. Without the use of rigid reinforcement in headgear, the fabric panels often deform undesirably and result in buckling and mismatching when placed on a patient's head. Some of these issues can be self-compensating by stretching due to the elasticity of the materials used; however, such stretching may cause undesirable pressure points and unwanted tensions, thus causing discomfort to the patient.

One solution to making better fitting, and thus more comfortable, headgear has been to stack one or more layers of different materials onto selective portions of the headgear in order to achieve desired variants in headgear properties. Stacking, however, results in a bulky and cumbersome headgear. Bulk has generally become a design restriction that limits the amount of customization and thus can leave a headgear underperforming.

US 2011/0253143 A2 refers to an adjustable headgear assembly for securing a respiratory interface device to the head of a patient. The headgear includes (i) a headgear member that fits over the crown and back portion of the head and to retention members on each side of the head which connect the headgear member with the patient interface device. According to one embodiment these retention members may be made of a different material than the headgear member fitting over the crown and back portion of the head. The retention members may, e.g. be made of a stretchable or non-stretchable fabric, such as made from waving, knitting, crocheting, knotting, felting or foam lamination. The retention members are in this case connected to the headgear member by means of adjustable couplings which may be accomplished through the use of a hook and loop fastening mechanism.

US 2008/0047560 A1 refers to a patient interface which includes a sealing arrangement that is adapted to provide an effective seal with the patient's nose, an inlet conduit arrangement adapted to deliver breathable gas to the sealing arrangement, and a cover that substantially encloses the sealing arrangement and/or the inlet conduit arrangement. The sealing arrangement as well as the inlet conduit arrangement may include two or more different materials with different properties (e.g. surface texture, hardness, thickness, etc.).

US 6,805,117 B1 refers to a universally fitting headgear that is readily adjustable to a variety of different sized patients and that couples a patient interface device to an airway of the patient. The headgear includes a headpiece that fits on the user's heal in a cap-like fashion.

### SUMMARY OF THE INVENTION

Accordingly, it is an object of the present invention to provide an improved material arrangement for use in a headgear for securing a patient interface device to the head of a patient that overcomes the shortcomings of conventional headgear. The present invention provides a means for changing the material properties of mask-mounting devices, thus allowing for the capability of ergonomic fit, pressure distribution, and additional support along a single layer. The present invention takes materials with different strengths and elasticises, pieces them together, and bonds them together on a single plane. By bonding different materials at specific locations on the same plane, varying areas of elasticity and strength are formed which accommodate the forces needed to secure a mask and evenly distribute pressures. Piecing the different materials on the same plane reduces bulk associated with some headgears. The ability to eliminate layers and bulk while fine tuning properties removes the current design restriction and allows for optimizing headgear performance.

This object is achieved according to one embodiment of the present invention which provides a method of forming a portion of a headgear assembly. The method comprises forming a plurality of sub-portions into generally planar, predetermined shapes, each of the sub-portions having at least one material property different than the other sub-portions and each sub-portion having a number of edge faces. The method further comprises coupling the plurality of sub-portions together in a predetermined orientation via their edge faces, wherein each of the coupled sub-portions are disposed in a common plane without an overlapping of the coupled sub-portions.

Coupling the sub-portions may comprise coupling the edge faces of the sub-portions, without overlap, by at least one of: stitching, adhering, heat bonding and sonic welding.

Coupling the sub-portions together may comprise coupling a layer of another of material to at least one of a top or bottom surface of each of the plurality of sub-portions.

This object is achieved according to another embodiment of the present invention which provides a material for use as a portion of a headgear assembly. The material comprises a first sub-portion formed from a generally planar first material and a second sub-portion formed from a generally planar second material. The second sub-portion is coupled along an edge face thereof to a corresponding edge face of the first sub-portion without overlapping the first sub-portion. The second material differs from the first material by at least one physical property.

The second sub-portion may be coupled to the first sub-portion via at least one of: stitching, adhering, heat bonding and sonic welding.

The material may further comprise a first layer of a third material coupled to at least one of a top face or a bottom face of each of the first and second sub-portions.

The material may further comprise a third sub-portion formed from a generally planar third material, the third sub-portion being coupled, without overlap, along an edge face to an edge face of at least one of the first sub-portion and the second sub-portion. The third material may differ from each of the first and second materials by at least one physical property.

The at least one physical property from which the first and second materials differ may be selected from the group consisting of elasticity, density, stiffness and breathability.

This object is achieved according yet another embodiment of the present invention which provides a headgear assembly comprising a first sub-portion formed from a generally planar first material and a second sub-portion formed from a generally planar second material. The second sub-portion is coupled along an edge face thereof to an edge face of the first sub-portion without overlapping the first sub-portion. The second material differs from the first material by at least one physical property.

The headgear assembly may further comprise a third sub-portion formed from a generally planar third material, the third sub-portion being coupled, without overlap, along an edge face thereof to an edge face of at least one of the first sub-portion and the second sub-portion, wherein the third material differs from each of the first and second materials by at least one physical property.

The headgear assembly may further comprise a fourth sub-portion formed from a generally planar fourth material which differs from each of the first, second, and third materials by at least one physical property. The fourth sub-portion being coupled, without overlap, along an edge face thereof to an edge face of at least one of the first sub-portion, the second sub-portion or the third sub-portion. The first sub-portion may comprise a back panel adapted to be disposed on a lower rear portion of a patient's head when the headgear assembly is disposed on the head of a patient. The fourth sub-portion may comprise a top strap adapted to be disposed at or about the top of a patient's head when the headgear assembly is disposed on the head of a patient. The second sub-portion may comprise a region disposed adjacent, and coupled to the top strap, the second sub-portion being adapted to secure the top strap in place on a patient's head when the headgear assembly is disposed on the head of a patient. The third sub-portion may comprise a curved member adapted to be disposed about, and maintain a designed angle around a patient's ear when the headgear assembly is disposed on the head of a patient. The first material may be highly elastic having a first elasticity, the second material may have a second elasticity less than the first elasticity, the third material may have a third elasticity less than the second elasticity, and the fourth material may have a fourth elasticity less than the first elasticity and the second elasticity.

The first sub-portion may comprise a generally c-shaped portion which is adapted to be disposed generally about a patient's ear to provide strength and prevent the headgear assembly from sagging or collapsing around the patient's ear when the headgear assembly is disposed on the head of a patient. The third sub portion may comprise a number of back straps adapted to be disposed on the back of a patient's head when the headgear assembly is disposed on the head of a patient. The second sub-portion may comprise a portion of a forward strap member adapted to be disposed below a patient's eye and to prevent the forward strap member from encroaching on the patient's eye when the headgear assembly is disposed on the head of a patient. The second sub-portion may further comprise another portion disposed adjacent, and coupled to, the c-shaped portion, the another portion being adapted to stabilize the number of back straps when the headgear assembly is disposed on the head of a patient. The first material may be a flexible, generally inelastic material, the second material may be less flexible than the first material, and the third material may be more elastic than the first material.

The third sub-portion may comprise a top strap adapted to be disposed at or about the top of a patient's head when the headgear assembly is disposed on the head of a patient and a pair of forward straps adapted to be disposed generally along a patient's cheek when the headgear assembly is disposed on the head of a patient. The second sub-portion may comprise a connecting member coupled between the pair of forward straps and a stabilizing portion disposed about the base of the top strap. The first sub-portion may comprise a plurality of back straps adapted to be disposed along the back of a patient's head when the headgear assembly is disposed on the head of a patient. The first material may have a first elasticity, the second material may have a second elasticity less than the first elasticity, and the third material may have a third elasticity less than the first elasticity and greater than the second elasticity.

The first sub-portion may comprise a number of portions adapted to be disposed about a patient's ears when the headgear assembly is disposed on the head of a patient, the number of portions being adapted to limit the overall elongation of the headgear assembly. The third sub-portion may comprise a top strap adapted to be disposed at or about the top of a patient's head when the headgear assembly is disposed on the head of a patient. The second sub-portion may comprise a first portion adapted to be disposed generally above a patient's ear when the headgear assembly is disposed on the head of a patient, the first portion being coupled between portions of the first sub-portion and to the top strap. The first portion being adapted to reduce the mobility of the top strap. The second sub-portion may further comprise a second portion adapted to be disposed forward of a patient's ear when the headgear assembly is disposed on the head of a patient, the second portion being coupled to portions of the first sub-portion. The first material may have a first elasticity, the second material may have a second elasticity less than the first elasticity, and the third material may have a third elasticity greater than the first elasticity and the second elasticity.

The headgear assembly may comprise a back panel adapted to be disposed on a rear portion of a patient's head when the headgear assembly is disposed on the head of a patient. The first sub-portion may comprise a central portion of the back panel. The second sub-portion may comprise a pair of secondary portions disposed on either side of the central portion. The third sub-portion may comprise a number of straps coupled to the secondary portions. The first material may have a first elasticity, the second material may be a generally stiff material having a second elasticity less than the first elasticity, and the third material may have a third elasticity less than the first elasticity and greater than the second elasticity.

### BRIEF DESCRIPTION OF THE DRAWINGS

FIG. 1A is an elevational view of a portion of a strap or other suitable portion of a headgear formed in accordance with an embodiment of the present invention;
FIG. 1B is an isometric view of the portion of FIG. 1A;
FIG. 2 is an isometric view of another portion of a strap or other suitable portion of a headgear formed in accordance with an embodiment of the present invention;
FIG. 3 is a side view of headgear incorporating portions formed in accordance with an embodiment of the present invention;
FIG. 4 is a side view of another headgear incorporating portions formed in accordance with an embodiment of the present invention;
FIGS. 5A and 5B, respectively, are side and rear views of a yet another headgear incorporating portions formed in accordance with an embodiment of the present invention;
FIG. 6 is a side view of a further headgear incorporating portions formed in accordance with an embodiment of the present invention; and
FIG. 7 is a rear view of yet a further headgear incorporating portions formed in accordance with an embodiment of the present invention.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

As used herein, the term "material" means an element formed from a single or layered (laminated) group of elements. For example, a typical laminate material used in headgear such as described herein includes a minimum of 3 layers such as: a top layer facing outwardly in UBL (unbreakable loop), a core formed from urethane foam which comes in various thickness and density to mandate the physical body (e.g., without limitation, 1.5 mm thick 10 lb/ft³ HyPUR-cel), and an inner layer of Lycra brand spandex which comes in various heaviness such as 10.5 oz and various spandex ratio to control the elasticity.

Directional phrases used herein, such as, for example and without limitation, top, bottom, left, right, upper, lower, front, back, and derivatives thereof, relate to the orientation of the elements shown in the drawings and are not limiting upon the claims unless expressly recited therein. Like numbers refer to like elements throughout.

As used herein, the terms "elastic" and "inelastic" shall be used to refer to the elasticity of a particular generally planar material generally within the plane of the material (i.e., in a direction perpendicular to an axis normal to the plane). As known in the art, the elasticity or inelasticity of a particular piece of planar material may be varied, for example, without limitation, by varying one or more of the density of a core material (e.g., foam) or by varying the content of spandex or other suitable elastic material in the planar material.

As used herein, the terms "rigid" and "flexible" shall be used to refer to the flexing or bending ability of a particular material. As known in the art, the rigidity or flexibility of a particular piece of planar material may be varied, for example, without limitation, by varying the density and/rigidity of a core material or by adding an additional generally rigid layer.

The present invention, which applicants have termed "Multi-Material Single-Plane Technology", changes the material properties of mask-mounting devices, e.g., without limitation, straps or headgear, allowing for the capability of ergonomic fit, pressure distribution, and additional support along a single layer. The present invention takes materials with different physical properties, e.g., without limitation, elasticities and stiffnesses, selectively arranges and bonds them together on a single plane. By bonding different materials at specific locations on the same plane, varying areas of elasticity and/or stiffness are selectively formed which accommodate the forces needed to secure a mask and evenly distribute pressures. Piecing the different materials together on the same plane reduces bulk associated with some headgears. The ability to eliminate layers and bulk while fine tuning properties removes current design restrictions and allows for optimization of headgear performance.

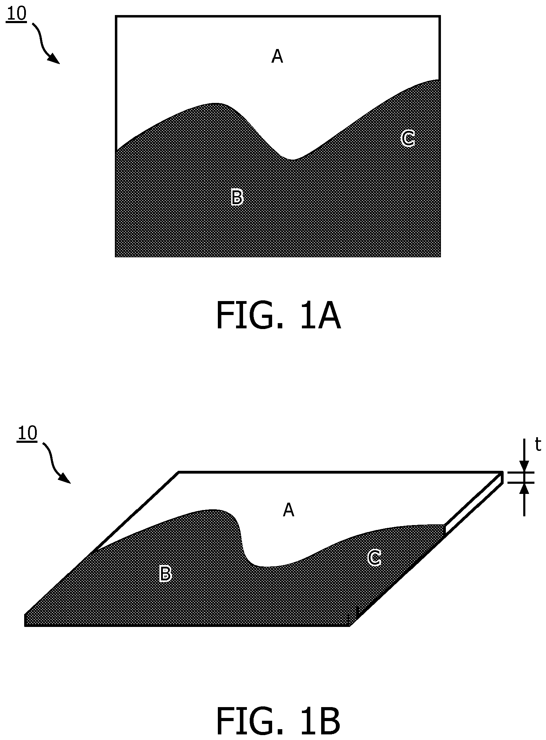

FIGS. 1A and 1B show an example of an embodiment of the present invention in the form of a selected region or portion 10 of a strap or other predetermined portion of a headgear assembly, such as for use in securing a patient interface device to the head of a patient (user), formed in accordance with the present invention. Portion 10 includes, and is formed from, three generally planar, different sections or sub-portions of a material or materials, denoted A, B and C (shown in different hatch patterns for contrast) that have been coupled together at their respective edge faces (not numbered), without overlap, to form a generally planar section of material. Such coupling of sub-portions A, B and C at their respective edge faces (i.e., abutment surfaces) may be accomplished for example, without limitation, through the use of stitching, adhesives, heat bonding, sonic welding, or other suitable means, without varying from the scope of the present invention.

Sub-portions A, B and C are preferably each formed from a respective material which differs in one or more physical properties from the adjacently bonded materials. Such physical properties may include, for example, without limitation, density, stiffness, elasticity, material orientation (e.g., a material which is elastic along an axis but generally inelastic along a different axis) breathability, or wicking ability. Such materials used may commonly be formed from laminated, woven, knit, or other suitable material and are preferably die cut to form the desired shapes prior to bonding.

As shown in FIG. 1B, sub-portions A, B and C are preferably bonded in a non-overlapping manner such that portion 10 is disposed generally in a single plane and is of a generally uniform thickness t. It is to be appreciated, however, that the thickness of different sub-portions A, B and C may vary slightly without varying from the scope of the present invention. It is also to be appreciated that neither the top or bottom surfaces of sub-portions A, B and C are necessarily disposed on the same plane, but instead the abutting edges of sub-portions A, B and C are coupled such that at least a portion of their thickness lie in the same plane.

The physical properties for each material used in one of sub-portions A, B and C is carefully selected such that the general properties of portion 10 may be custom tailored to fit a particular need, as will be appreciated from the further example embodiments described herein. It is to be appreciated that while three different sub-portions A, B and C are shown in the example of FIGS. 1A and 1B, the present invention contemplates that the quantity and shape of sub-portions that are coupled together to form a portion 10 having desired physical characteristics may be varied without varying from the scope of the present invention.

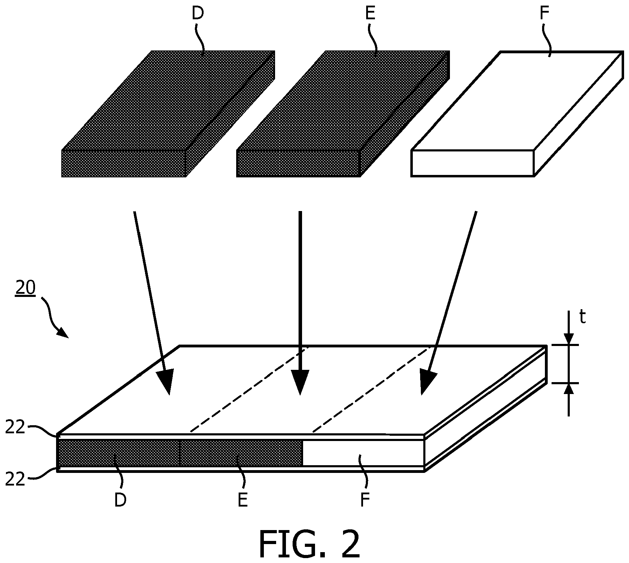

FIG. 2 shows another example embodiment of the present invention in the form of a portion 20 of a strap or other predetermined portion of a headgear assembly, wherein sub-portions D, E and F (shown both exploded from, and coupled in, portion 20 with different hatching for clarity) have been held together in a single plane through a lamination process, wherein one or more layers 22 (two are employed in the embodiment of FIG. 2) of a fabric or other suitable material, (e.g., without limitation Lycra (fabric), silicone) secures the different sub portions D, E and F in the single plane by being bonded to at least one of the top and bottom surfaces (not numbered) of each of sub-portions D, E and F (i.e., the surfaces that are perpendicular to the abutment surfaces).

It is to be appreciated that sub-portions D, E and F, like sub-portions A, B and C, previously discussed, may be formed from single elements or from multiple elements laminated or otherwise coupled together to form each of sub-portions D, E and F. The laminated arrangement one or more layers 22 may be the sole means coupling sub portions D, E and F or may also be done in addition to coupling of the edge (abutment) faces of sub-portions D, E and F, such as previously described in reference to FIGS. 1A and 1B. Like portion 10 previously discussed, portion 20 is of a generally uniform thickness t, although slight variations in the thickness may occur without varying from the scope of the present invention. Also like portion 10 previously discussed, sub-portions D, E and F of portion 20 are each preferably formed from a respective material which differs in one or more physical properties from the adjacent materials. Such physical properties may include, for example, without limitation, density, stiffness, elasticity, material orientation (e.g., a material which is elastic along an axis but generally inelastic along a second axis), breathability, or wicking ability. Preferably, layers 22 are each of sufficient pliability/elasticity so as to not mask the varying material properties of sub-portions D, E and F coupled thereto.

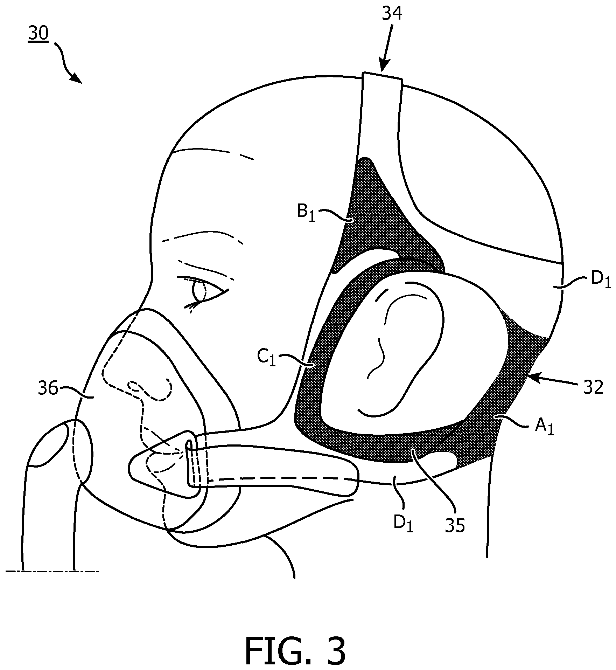

Having thus described the basic concepts of the invention, application of such concepts in a number of example applications will now be provided in conjunction with FIGS. 3, 4, 5A-B, 6 and 7. It is to be appreciated that customizing selected portions of the headgear by coupling different structural materials without overlap as described herein can be applied to a number of headgear applications to improve fit, comfort, and efficacy while using a minimal amount of material, thus avoiding bulk in the headgear. It is to be appreciated that the techniques and concepts described herein can be used to modify generally any soft headgear into a more ergonomic form. By selectively manipulating the physical properties of selected portions of a headgear, such portions are better able to conform to the shape or contour of the corresponding anatomic area of interest on the user's head and thus provide a more ergonomic fit. Such potential areas of concern may include, for example, without limitation, around the cheek bone, the base of the skull/occipital area, the crown of the head, chin, and temporal/parietal curve around the ear.

FIG. 3 shows a side view of an example headgear assembly 30 formed in accordance with an embodiment of the present invention disposed on the head of a patient. Headgear assembly 30 utilizes four different materials A₁, B₁, C₁, D₁ (shown in different hatch patterns for clarity) which vary in at least one of elasticity and/or stiffness, shaped and coupled together without overlap (as previously described) in a predetermined arrangement. Headgear assembly 30 includes a back panel 32 adapted to be disposed on a lower rear portion of a patient's head when headgear assembly 30 is disposed on the head of a patient. Back panel 32 is formed from a highly elastic first material A₁ (e.g., without limitation, a laminate material having a low density foam with a Lycra backing in highest spandex ratio) which allows for increased comfort and fit when the patient's head pivots back and forth. A generally rigid, less elastic (in comparison to first material A₁), second material B₁ is adapted to be disposed generally above a patient's ear and functions as generally as a keystone, securing a top strap 34 (which is coupled thereto) of headgear assembly 30 in place when headgear assembly 30 is disposed on the head of a patient.

Headgear assembly 30 further includes a curved member 35 formed from a third material C₁ (e.g., without limitation, dense foam having a less or non-elastic backing) having a low, if any, elasticity, less than the elasticity of second material B₁. Curved member 35 serves to maintain the designed angle around the patient's ear as a patient interface, such as mask 36, is tightened on the patient's head. Top strap 34, as well as the remainder of headgear assembly 30, is formed from a generally elastic fourth material D₁ (e.g., without limitation, a typical strapping material having an elongation in the range of about 125% to 150%) having an elasticity less than the second material B₁ and first material A₁. It is to be appreciated that the four different materials (coupled edge to edge as described in the example of FIGS 1A and 1B) act to provide specific individual functions in the specific regions where they are positioned. In contrast, conventional stacking of materials to provide similar properties would create an overly bulky headgear which would be less desirable to a patient, especially in applications where such headgear would need to be worn while laying down.

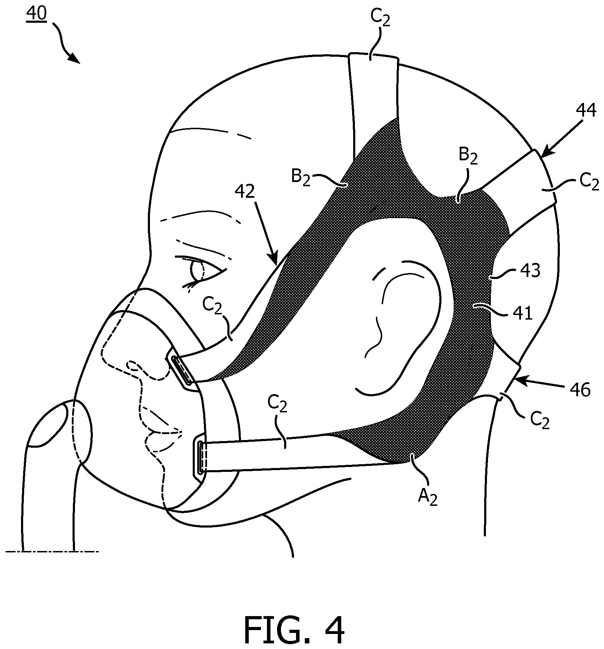

FIG. 4 shows a side view of another example headgear assembly 40 formed in accordance with an embodiment of the present invention disposed on the head of a patient. Headgear 40 utilizes three different materials A₂, B₂, C₂ (shown in different hatch patterns for clarity) which vary in at least one of elasticity and/or stiffness, shaped and coupled together without overlap (as previously described) in a predetermined arrangement. A flexible, minimally elastic, preferably inelastic, first material A₂ (e.g., without limitation, a foam core material with a non-elastic backing) forms a generally c-shaped portion 41 which is adapted to be disposed generally about a patient's ear to provide strength and prevent headgear assembly 40 from sagging or collapsing around the patient's ear when headgear assembly 40 is disposed on the head of a patient.

A more stiff, preferably nearly rigid, second material B₂ (e.g., a layered material including a rigid polymer sheet) is coupled to c-shaped portion 41 and forms a portion of a forward strap member 42 of headgear assembly 40 and acts to keep strap member 42 from encroaching on the patient's eye. Another portion 43 of second material B₂ is disposed adjacent, and coupled to a rear edge (not numbered) of c-shaped portion 41 and is used to stabilize back straps 44 and 46. A third, generally elastic and flexible material C₂ (e.g., a material having an elongation in the range of about 125% to 150%), not hatched, forms back straps 44 and 46 (which are each coupled to portion 43), a top strap 47, as well as the remainder of headgear 40.

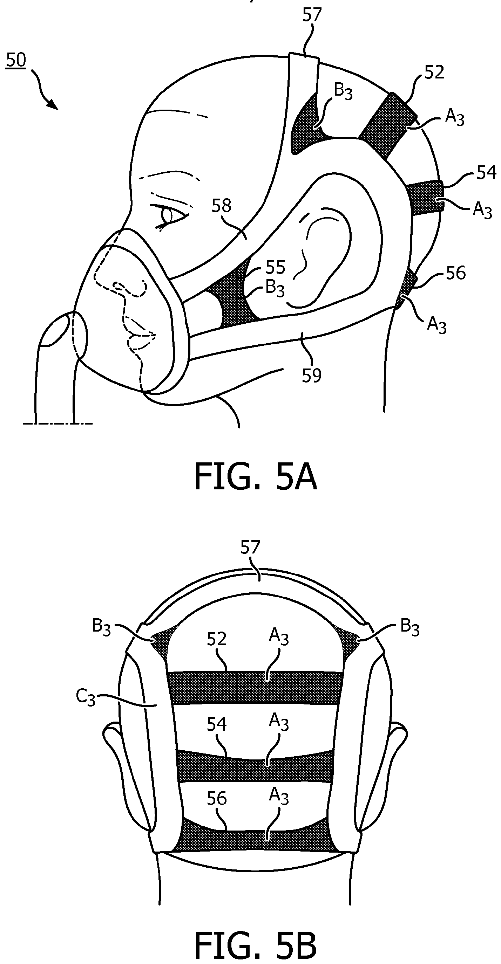

FIGS. 5A and 5B, respectively, show side and back views of yet another example headgear assembly 50 formed in accordance with an embodiment of the present invention disposed on the head of a patient. Headgear 50 utilizes three different materials A₃, B₃, C₃ (shown in different hatch patterns for clarity) which vary in at least one of elasticity and/or stiffness, shaped and coupled together (as previously described) in a predetermined arrangement. Headgear assembly 50 includes a number of back straps 52, 54, 56 formed from a highly elastic first material A₃ (e.g., without limitation, a laminate material having a low density foam with a Lycra backing in highest spandex ratio) which allows for conformation around anatomical areas of the patient's head.

Headgear assembly 50 further includes a connecting member 55 formed from a generally inelastic, yet flexible, second material B₃ which spans between a pair of forward strap members 58, 59. The inelasticity of connecting member 55 acts to keep strap members 58 and 59 in a relatively parallel arrangement in order to provide optimal strapping force vectors when headgear assembly 50 is disposed on the head of a patient. Generally inelastic second material B₃ also forms a stabilizing portion (not numbered) disposed about the base of a top strap 57 in order to restrict mobility of top strap 57. Top strap 57 and forward strap members 58,59 are formed from a third material C₃ (not hatched) having an elasticity somewhere between first and second materials A₃ and B₃ (e.g., a material having an elongation in the range of about 125% to 150). The remainder of headgear assembly 50 is also generally formed from third material C₃.

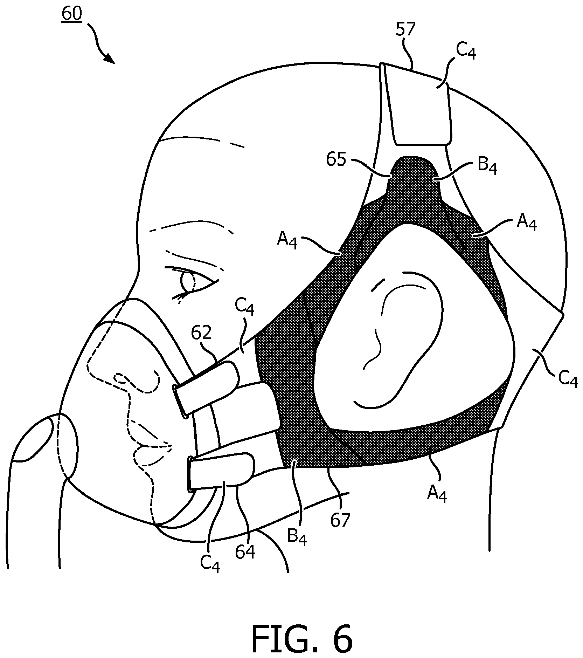

FIG. 6 shows a side view of a further example headgear assembly 60 formed in accordance with an embodiment of the present invention disposed on the head of a patient. Headgear 60 utilizes three different materials A₄, B₄, C₄ (shown in different hatch patterns for clarity) which vary in at least one of elasticity and/or stiffness, shaped and coupled together (as previously described) in a predetermined arrangement. Headgear assembly 60 is formed in part from a flexible, somewhat elastic first material A₄ disposed in selected portions about the patient's ear which act to limit the overall elongation of headgear assembly 60 and reduces the length of straps adjustment 62, 64 needed for adjustment. Headgear assembly 60 further includes a first portion 65 formed from a generally stiff, generally inelastic (less elastic than first material A₄), second material B₄ which is adapted to be disposed generally above a patient's ear and is coupled between portions of first material A₄.

First portion 65 serves to reduce the mobility of a top strap 66 and also to maintain comfortable eye proximity for headgear assembly 60 while allowing for adequate mask tightening when headgear assembly 60 is disposed on the head of a patient. Headgear assembly 60 also includes a second portion 67 formed from second material B₄ which is adapted to be disposed forward of a patient's ear when headgear assembly 60 is disposed on the head of a patient and is coupled to portions of first material A₄. Top strap 66, as well as the remainder of headgear assembly 60, is formed from a generally elastic, third material C₄ (e.g., a material having an elongation in the range of about 125% to 150), which is less stiff than second material B₄ (not hatched).

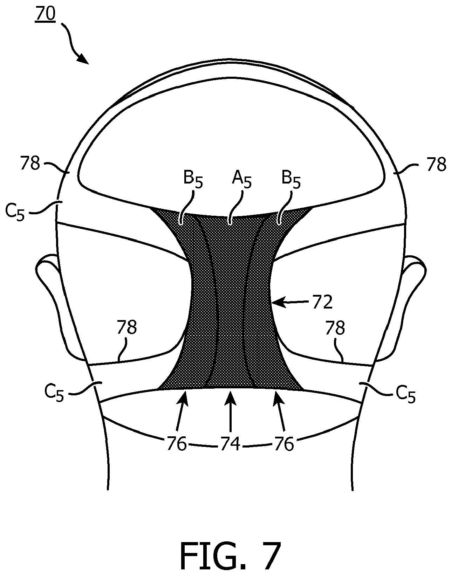

FIG. 7 shows a rear view of a another example headgear assembly 70 formed in accordance with an embodiment of the present invention disposed on the head of a patient. Headgear 70 includes a back panel 72 which utilizes three different materials A₅, B₅, C₅ (shown in different hatch patterns for clarity), which vary in at least one of elasticity and/or stiffness, shaped and coupled together (as previously described) in a predetermined arrangement. The use of different materials A₅, B₅ and C₅ provides support for the back panel 72 and reduces the amount of stretch seen in this area. Back panel 72 includes a central portion 74 formed from a highly elastic first material A₅ and further includes a pair of secondary portions 76 each formed from a relatively rigid second material B₅ coupled on either side of central portion 74. In use, secondary portions 76 serve to prevent collapse of back panel 72 while central portion 74 assists in fitting of back panel 72 to the rear of a patient's head and allows for folding of back panel when headgear assembly 70 is not installed on the head of a patient. A flexible, slightly elastic, third material C₅ (not hatched) forms a number of straps 78 which couple back panel 72 to the remainder of headgear assembly 70.

It can be appreciated from the foregoing examples that the present invention provides improvements to headgear or portions thereof These improvements enhance the fit and stability, and thus the comfort of the headgear when worn by a user. In particular, the present invention provides for selected portions of a headgear to remain securely placed in desired locations, thus reducing undesirable unevenness in tensions throughout the headgear. It is to be understood that other arrangements beyond those particularly described in the examples herein may be employed without varying from the scope of the present invention.

It is also to be understood that the present invention contemplates the use of various materials including, but not limited to, those particularly cited herein. Velstretch and spandex are two non-limiting examples of suitable elastic materials. Polyurethane, silicone, and certain foams are non-limiting examples of suitable semi-elastic materials. Mylar, polyethylene, Nylon, UBL, and various dense foams are non-limiting examples of suitable inelastic materials. It is to be appreciated that the particular materials identified herein are provided for example purposes only, and are not intended to be limiting upon the scope of the present invention.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A method of forming a portion (10) of a headgear assembly (30, 40, 50, 60, 70), the method comprising:
forming a plurality of sub-portions (A, B, C, A₂, B₂, C₂, A₃, B₃, C₃, A₄, B₄, C₄, A₅, B₅, C₅) into generally planar, predetermined shapes, each of the sub-portions having at least one material property different than the other sub-portions and each sub-portion having a number of edge faces;
**characterized in** the step of:
coupling the plurality of sub-portions together in a predetermined orientation via their edge faces, wherein each of the coupled sub-portions are disposed in a common plane without an overlapping of the coupled sub-portions.

2. The method of claim 1 wherein coupling the sub-portions comprises coupling the edge faces of the sub-portions, without overlap, by at least one of: stitching, adhering, heat bonding and sonic welding.

3. The method of claim 1 wherein coupling the sub-portions together comprises coupling a layer of another of material to at least one of a top or bottom surface of each of the plurality of sub-portions.

4. A material for use as a portion (10) of a headgear assembly (30, 40, 50, 60, 70), the material comprising:
a first sub-portion (A) formed from a generally planar first material; and
a second sub-portion (B) formed from a generally planar second material,
wherein the second material differs from the first material by at least one physical property,
**characterized in that** the second sub-portion is coupled along an edge face thereof to a corresponding edge face of the first sub-portion without overlapping the first sub-portion.

5. The material of claim 4 wherein the second sub-portion is coupled to the first sub-portion via at least one of: stitching, adhering, heat bonding and sonic welding.

6. The material of claim 4 further comprising a first layer of a third material coupled to at least one of a top face or a bottom face of each of the first and second sub-portions.

7. The material of claim 4 further comprising a third sub-portion (C) formed 10 from a generally planar third material, the third sub-portion being coupled, without overlap, along an edge face to an edge face of at least one of the first sub-portion and the second sub-portion, wherein the third material differs from each of the first and second materials by at least one physical property.

8. The material of claim 4 wherein the at least one physical property from which the first and second materials differ is selected from the group consisting of elasticity, density, stiffness and breathability.

9. A headgear assembly comprising:
a first sub-portion formed from a generally planar first material; and
a second sub-portion formed from a generally planar second material,
wherein the second material differs from the first material by at least one physical property,
**characterized in that** the second sub-portion is coupled along an edge face thereof to an edge face of the first sub-portion without overlapping the first sub-portion.

10. The headgear assembly of claim 9 further comprising a third sub-portion formed from a generally planar third material, the third sub-portion being coupled, without overlap, along an edge face thereof to an edge face of at least one of the first sub-portion and the second sub-portion, wherein the third material differs from each of the first and second materials by at least one physical property.

11. The headgear assembly (30) of claim 10 further comprising a fourth sub-portion formed from a generally planar fourth material which differs from each of the first, second, and third materials by at least one physical property, the fourth sub-portion being coupled, without overlap, along an edge face thereof to an edge face of at least one of the first sub-portion, the second sub-portion or the third sub-portion, wherein:
the first sub-portion (A₁) comprises a back panel (32) adapted to be disposed on a lower rear portion of a patient's head when the headgear assembly is disposed on the head of a patient;
the fourth sub-portion (D₁) comprises a top strap (34) adapted to be disposed at or about the top of a patient's head when the headgear assembly is disposed on the head of a patient;
the second sub-portion (B₁) comprises a region disposed adjacent, and coupled to the top strap, the second sub-portion being adapted to secure the top strap in place on a patient's head when the headgear assembly is disposed on the head of a patient;
the third sub-portion (C₁) comprises a curved member (35) adapted to be disposed about, and maintain a designed angle around, a patient's ear when the headgear assembly is disposed on the head of a patient;
the first material is highly elastic having a first elasticity;
the second material has a second elasticity less than the first elasticity;
the third material has a third elasticity less than the second elasticity; and
the fourth material has a fourth elasticity less than the first elasticity and the second elasticity.

12. The headgear assembly (40) of claim 10 wherein:
the first sub-portion (A₂) comprises a generally c-shaped portion (41) which is adapted to be disposed generally about a patient's ear to provide strength and prevent the headgear assembly from sagging or collapsing around the patient's ear when the headgear assembly is disposed on the head of a patient;
the third sub portion (C₃) comprises a number of back straps (44, 46) adapted to be disposed on the back of a patient's head when the headgear assembly is disposed on the head of a patient;
the second sub-portion (B₂) comprises:
a portion of a forward strap member (42) adapted to be disposed below a patient's eye and is adapted to prevent the forward strap member from encroaching on the patient's eye when the headgear assembly is disposed on the head of a patient, and
another portion 43 disposed adjacent, and coupled to, the c-shaped portion, the another portion being adapted to stabilize the number of back straps when the headgear assembly is disposed on the head of a patient;
the first material is a flexible, generally inelastic material;
the second material is less flexible than the first material; and
the third material is more elastic than the first material.

13. The headgear assembly (50) of claim 10 wherein:
the third sub-portion (C₃) comprises:
a top strap (57) adapted to be disposed at or about the top of a patient's head when the headgear assembly is disposed on the head of a patient, and
a pair of forward straps (58, 59) adapted to be disposed generally along a patient's cheek when the headgear assembly is disposed on the head of a patient;
the second sub-portion (B₃) comprises:
a connecting member (55) coupled between the pair of forward straps,
and
a stabilizing portion disposed about a base of the top strap;
the first sub-portion (A₃) comprises a plurality of back straps (52, 54, 56) adapted to be disposed along the back of a patient's head when the headgear assembly is disposed on the head of a patient;
the first material has a first elasticity;
the second material has a second elasticity less than the first elasticity; and
the third material has a third elasticity less than the first elasticity and greater than the second elasticity.

14. The headgear assembly (60) of claim 10 wherein:
the first sub-portion (A₄) comprises a number of portions adapted to be disposed about a patient's ears when the headgear assembly is disposed on the head of a patient, the number of portions being adapted to limit the overall elongation of the headgear assembly;
the third sub-portion comprises a top strap (66) adapted to be disposed at or about the top of a patient's head when the headgear assembly is disposed on the head of a patient;
the second sub-portion (B₄) comprises:
a first portion (65) adapted to be disposed generally above a patient's ear when the headgear assembly is disposed on the head of a patient, the first portion being coupled between portions of the first sub-portion and to the top strap, the first portion being adapted to reduce the mobility of the top strap, and
a second portion (67) adapted to be disposed forward of a patient's ear when the headgear assembly is disposed on the head of a patient, the second portion being coupled to portions of the first sub-portion;
the first material has a first elasticity;
the second material has a second elasticity less than the first elasticity; and
the third material has a third elasticity greater than the first elasticity and the second elasticity.

15. The headgear assembly (70) of claim 10 wherein:
the headgear assembly comprises a back panel (72) adapted to be disposed on a rear portion of a patient's head when the headgear assembly is disposed on the head of a patient;
the first sub-portion (A₅) comprises a central portion (74) of the back panel;
the second sub-portion (B₅) comprises a pair of secondary portions (76) disposed on either side of the central portion;
the third sub-portion (C₅) comprises a number of straps (78) coupled to the secondary portions;
the first material has a first elasticity;
the second material is a generally stiff material having a second elasticity less than the first elasticity; and
the third material has a third elasticity less than the first elasticity and greater than the second elasticity.

## Patentansprüche

1. Verfahren zum Formen eines Abschnitts (10) einer Kopfgeschirrbaugruppe (30, 40, 50, 60, 70), wobei das Verfahren Folgendes umfasst:
Formen einer Vielzahl von Teilabschnitten (A, B, C, A₂, B₂, C₂, A₃, B₃, C₃, A₄, B₄, C₄, A₅, B₅ C₅) zu im Allgemeinen flachen, vordefinierten Formen, wobei sich jeder der Teilabschnitte in mindestens einer Materialeigenschaft von den anderen Teilabschnitten unterscheidet und wobei jeder Teilabschnitt eine Anzahl von Stirnflächen hat;
**gekennzeichnet durch** den Schritt des:
Koppelns der Vielzahl von Teilabschnitten miteinander in einer vorgegebenen Orientierung über ihre Stirnflächen, wobei jeder der gekoppelten Teilabschnitte in einer gemeinsamen Ebene angeordnet ist, ohne dass sich die gekoppelten Teilabschnitte überlappen.

2. Verfahren nach Anspruch 1, wobei das Koppeln der Teilabschnitte das Koppeln der Stirnflächen der Teilabschnitte ohne Überlappung durch mindestens eines von: Nähen, Kleben, Heißverkleben und Ultraschallschweißen umfasst

3. Verfahren nach Anspruch 1, wobei das Koppeln der Teilabschnitte miteinander das Koppeln einer Schicht aus einem anderen Material mit mindestens einer oberen oder unteren Fläche von jedem der Vielzahl von Teilabschnitten umfasst.

4. Material zur Verwendung als ein Abschnitt (10) einer Kopfgeschirrbaugruppe (30, 40, 50, 60, 70), wobei das Material Folgendes umfasst:
einen ersten Teilabschnitt (A), der aus einem im Allgemeinen flachen ersten Material geformt ist; und
einen zweiten Teilabschnitt (B), der aus einem im Allgemeinen flachen zweiten Material geformt ist,
wobei sich das zweite Material in mindestens einer physikalischen Eigenschaft von dem ersten Material unterscheidet,
**dadurch gekennzeichnet, dass** der zweite Teilabschnitt entlang einer Stirnfläche hiervon mit einer übereinstimmenden Stirnfläche des ersten Teilabschnitts gekoppelt ist, ohne den ersten Teilabschnitt zu überlappen.

5. Material nach Anspruch 4, wobei der zweite Teilabschnitt über mindestens eines von: Nähen, Kleben, Heißverkleben und Ultraschallschweißen mit dem ersten Teilabschnitt gekoppelt ist.

6. Material nach Anspruch 4, das weiterhin eine erste Schicht aus einem dritten Material umfasst, das mit mindestens einer oberen Fläche oder einer unteren Fläche von jedem, dem ersten und dem zweiten Teilabschnitt gekoppelt ist.

7. Material nach Anspruch 4, das weiterhin einen dritten Teilabschnitt (C) umfasst, welcher aus einem im Allgemeinen flachen dritten Material geformt ist, wobei der dritte Teilabschnitt ohne Überlappung entlang einer Stirnfläche mit der Stirnfläche von mindestens einem von dem ersten Teilabschnitt und dem zweiten Teilabschnitt gekoppelt ist, wobei sich das dritte Material in mindestens einer physikalischen Eigenschaft von jedem, dem ersten und dem zweiten Material unterscheidet.

8. Material nach Anspruch 4, wobei die mindestens eine physikalische Eigenschaft, durch die sich das erste und das zweite Material unterscheiden, ausgewählt wird aus der Gruppe bestehend aus Elastizität, Dichte, Steifheit und Atmungsaktivität.

9. Kopfgeschirrbaugruppe, die Folgendes umfasst:
einen ersten Teilabschnitt, der aus einem im Allgemeinen flachen ersten Material geformt ist; und
einen zweiten Teilabschnitt, der aus einem im Allgemeinen flachen zweiten Material geformt ist,
wobei sich das zweite Material in mindestens einer physikalischen Eigenschaft von dem ersten Material unterscheidet,
**dadurch gekennzeichnet, dass** der zweite Teilabschnitt entlang einer Stirnfläche hiervon mit einer Stirnfläche des ersten Teilabschnitts gekoppelt ist, ohne den ersten Teilabschnitt zu überlappen.

10. Kopfgeschirrbaugruppe nach Anspruch 9, weiterhin umfassend einen dritten Teilabschnitt, der aus einem im Allgemeinen flachen dritten Material geformt ist, wobei der dritte Teilabschnitt ohne Überlappung entlang einer Stirnfläche hiervon mit einer Stirnfläche von mindestens einem von dem ersten Teilabschnitt und dem zweiten Teilabschnitt gekoppelt ist, wobei sich das dritte Material in mindestens einer physikalischen Eigenschaft von jedem, dem ersten und dem zweiten Material unterscheidet.

11. Kopfgeschirrbaugruppe (30) nach Anspruch 10, weiterhin umfassend einen vierten Teilabschnitt, der aus einem im Allgemeinen flachen vierten Material geformt ist, der sich in mindestens einer physikalischen Eigenschaft von jedem, dem ersten, zweiten und dritten Material unterscheidet, wobei der vierte Teilabschnitt ohne Überlappung entlang einer Stirnfläche hiervon mit einer Stirnfläche von mindestens einem, dem ersten Teilabschnitt, dem zweiten Teilabschnitt oder dem dritten Teilabschnitt gekoppelt ist, wobei:
der erste Teilabschnitt (A₁) eine Rückwand (32) umfasst, die dafür ausgelegt ist, an einem unteren rückseitigen Bereich des Patientenkopfs angeordnet zu werden, wenn die Kopfgeschirrbaugruppe am Kopf eines Patienten angeordnet wird;
der vierte Teilabschnitt (D₁) einen oberen Riemen (34) umfasst, der dafür ausgelegt ist, an oder um den oberen Teil eines Patientenkopfs angeordnet zu werden, wenn die Kopfgeschirrbaugruppe am Kopf eines Patienten angeordnet wird;
der zweite Teilabschnitt (B₁) einen Bereich umfasst, der angrenzend an den oberen Riemen angeordnet und hiermit gekoppelt ist, wobei der zweite Teilabschnitt dafür ausgelegt ist, den oberen Riemen am Patientenkopf in seiner Position zu halten, wenn die Kopfgeschirrbaugruppe am Kopf eines Patienten angeordnet wird;
der dritte Teilabschnitt (C₁) ein gekrümmtes Element (35) umfasst, das dafür ausgelegt ist, um ein Patientenohr herum angeordnet zu werden und einen bestimmten Winkel um das Patientenohr aufrechtzuerhalten, wenn die Kopfgeschirrbaugruppe am Kopf eines Patienten angeordnet wird;
das erste Material hochelastisch ist und eine erste Elastizität aufweist;
das zweite Material eine zweite Elastizität aufweist, die geringer ist als die erste Elastizität;
das dritte Material eine dritte Elastizität aufweist, die geringer als die zweite Elastizität ist; und
das vierte Material eine vierte Elastizität aufweist, die geringer als die erste Elastizität und die zweite Elastizität ist.

12. Kopfgeschirrbaugruppe (40) nach Anspruch 10, wobei:
der erste Teilabschnitt (A₂) einen im Allgemeinen c-förmigen Abschnitt (41) umfasst, der dafür ausgelegt ist, im Allgemeinen um ein Patientenohr herum angeordnet zu werden, um für Festigkeit zu sorgen und zu verhindern, dass sich die Kopfgeschirrbaugruppe um das Patientenohr herum absenkt oder zusammenfällt, wenn die Kopfgeschirrbaugruppe am Kopf eines Patienten angeordnet wird;
der dritte Teilabschnitt (C₂[Editor1]) eine Anzahl von rückseitigen Riemen (44, 46) umfasst, die dafür ausgelegt sind, an der Rückseite des Patientenkopfs angeordnet zu werden, wenn die Kopfgeschirrbaugruppe am Kopf eines Patienten angeordnet wird;
der zweite Teilabschnitt (B₂) Folgendes umfasst:
einen Abschnitt eines vorderen Riemenelements (42), der dafür ausgelegt ist, unterhalb eines Patientenauges angeordnet zu werden, und der verhindern soll, dass das vordere Riemenelement das Patientenauge beeinträchtigt, wenn die Kopfgeschirrbaugruppe am Kopf eines Patienten angeordnet wird, und
einen weiteren Abschnitt (43), der angrenzend an den c-förmigen Abschnitt angeordnet und hiermit gekoppelt ist, wobei der weitere Abschnitt dafür ausgelegt ist, die Anzahl von rückseitigen Riemen zu stabilisieren, wenn die Kopfgeschirrbaugruppe am Kopf eines Patienten angeordnet wird;
das erste Material ein flexibles, im Allgemeinen nicht elastisches Material ist;
das zweite Material weniger flexibel ist als das erste Material; und
das dritte Material elastischer ist als das erste Material.

13. Kopfgeschirrbaugruppe (50) nach Anspruch 10, wobei:
der dritte Teilabschnitt (C₃) Folgendes umfasst:
einen oberen Riemen (57), der dafür ausgelegt ist, an oder um den oberen Bereich eines Patientenkopfs angeordnet zu werden, wenn die Kopfgeschirrbaugruppe am Kopf eines Patienten angeordnet wird, und
ein Paar von vorderen Riemen (58, 59), die dafür ausgelegt sind, im Allgemeinen entlang einer Patientenwange angeordnet zu werden, wenn die Kopfgeschirrbaugruppe am Kopf eines Patienten angeordnet wird;
der zweite Teilabschnitt (B₃) Folgendes umfasst:
ein Verbindungselement (55), das zwischen das vordere Riemenpaar gekoppelt ist, und
einen stabilisierenden Abschnitt, der um eine Basis des oberen Riemens herum angeordnet ist;
der erste Teilabschnitt (A₃) eine Vielzahl von rückseitigen Riemen (52, 54, 56) umfasst, die dafür ausgelegt sind, entlang der Rückseite des Patientenkopfs angeordnet zu werden, wenn die Kopfgeschirrbaugruppe am Kopf eines Patienten angeordnet wird;
das erste Material eine erste Elastizität aufweist;
das zweite Material eine zweite Elastizität aufweist, die geringer als die erste Elastizität ist; und
das dritte Material eine dritte Elastizität aufweist, die geringer als die erste Elastizität und größer als die zweite Elastizität ist.

14. Kopfgeschirrbaugruppe (60) nach Anspruch 10, wobei
der erste Teilabschnitt (A₄) eine Vielzahl von Abschnitten umfasst, die dafür ausgelegt sind, um ein Patientenohr herum angeordnet zu werden, wenn die Kopfgeschirrbaugruppe am Kopf eines Patienten angeordnet wird, wobei die Vielzahl von Abschnitten dafür ausgelegt sind, die Gesamtdehnung des Kopfgeschirrbaugruppe zu begrenzen;
der dritte Teilabschnitt einen oberen Riemen (66) umfasst, der dafür ausgelegt ist, an oder um den oberen Bereich eines Patientenkopfs herum angeordnet zu werden, wenn die Kopfgeschirrbaugruppe am Kopf eines Patienten angeordnet wird;
der zweite Teilabschnitt (B₄) Folgendes umfasst:
einen ersten Abschnitt (65), der dafür ausgelegt ist, im Allgemeinen über einem Patientenohr angeordnet zu werden, wenn die Kopfgeschirrbaugruppe am Kopf eines Patienten angeordnet wird, wobei der erste Abschnitt zwischen Abschnitte des ersten Teilabschnitts und mit dem oberen Riemen gekoppelt wird, wobei der erste Abschnitt dafür ausgelegt ist, die Beweglichkeit des oberen Riemens zu reduzieren, und
einen zweiten Abschnitt (67), der dafür ausgelegt ist, vor einem Patientenohr angeordnet zu werden, wenn die Kopfgeschirrbaugruppe am Kopf eines Patienten angeordnet wird, wobei der zweite Abschnitt mit Abschnitten des ersten Teilabschnitts gekoppelt wird;
das erste Material eine erste Elastizität aufweist;
das zweite Material eine zweite Elastizität aufweist, die geringer als die erste Elastizität ist; und
das dritte Material eine dritte Elastizität aufweist, die größer als die erste Elastizität und die zweite Elastizität ist.

15. Kopfgeschirrbaugruppe (70) nach Anspruch 10, wobei
die Kopfgeschirrbaugruppe eine Rückwand (72) umfasst, die dafür ausgelegt ist, am hinteren Bereich eines Patientenkopfs angeordnet zu werden, wenn die Kopfgeschirrbaugruppe am Kopf eines Patienten angeordnet wird;
der erste Teilabschnitt (A₅) einen mittleren Abschnitt (74) der Rückwand umfasst;
der zweite Teilabschnitt (B₅) ein Paar von sekundären Abschnitten (76) umfasst, die auf beiden Seiten des mittleren Abschnitts angeordnet sind;
der dritte Teilabschnitt (C₅) eine Anzahl von Riemen (78) umfasst, die mit den sekundären Abschnitten gekoppelt sind;
das erste Material eine erste Elastizität aufweist;
das zweite Material ein im Allgemeinen steifes Material mit einer zweiten Elastizität ist, die geringer als die erste Elastizität ist; und
das dritte Material eine dritte Elastizität aufweist, die geringer als die erste Elastizität und größer als die zweite Elastizität ist.

## Revendications

1. Procédé de formation d'une partie (10) d'un ensemble casque (30, 40, 50, 60, 70), le procédé comprenant :
la formation d'une pluralité de sous-parties (A, B, C, A₂, B₂, C₂, A₃, B₃, C₃, A₄, B₄, C₄, A₅, B₅, C₅) en des formes prédéterminées généralement planes, chacune des sous-parties possédant au moins une propriété de matériau différente des autres sous-parties et chaque sous-partie possédant un certain nombre de faces de bord ;
**caractérisé par** l'étape consistant à :
coupler la pluralité de sous-parties les unes aux autres dans une orientation prédéterminée par l'intermédiaire de leurs faces de bord, dans lequel chacune des sous-parties couplées est disposée dans un plan commun sans se chevauchant des sous-parties couplées.

2. Procédé selon la revendication 1, dans lequel le couplage des sous-parties comprend le couplage des faces de bord des sous-parties, sans chevauchement, par au moins une technique parmi : couture, adhésion, liaison par la chaleur et soudage sonique.

3. Procédé selon la revendication 1, dans lequel le couplage des sous-parties les unes aux autres comprend le couplage d'une couche d'un autre matériau à au moins l'une parmi une surface supérieure ou inférieure de chacune parmi la pluralité de sous-parties.

4. Matériau pour une utilisation en tant que partie (10) d'un ensemble casque (30, 40, 50, 60, 70), le matériau comprenant :
une première sous-partie (A) formée à partir d'un premier matériau généralement plan ; et
une deuxième sous-partie (B) formée à partir d'un deuxième matériau généralement plan, dans lequel le deuxième matériau diffère du premier matériau par au moins une propriété physique,
**caractérisé en ce que** la deuxième sous-partie est couplée le long d'une face de bord de celle-ci à une face de bord correspondante de la première sous-partie sans chevaucher la première sous-partie.

5. Matériau selon la revendication 4, dans lequel la deuxième sous-partie est couplée à la première sous-partie par l'intermédiaire d'au moins une technique parmi :
couture, adhésion, liaison par la chaleur et soudage sonique.

6. Matériau selon la revendication 4, comprenant en outre une première couche d'un troisième matériau, couplée à au moins l'une parmi une face supérieure ou une face inférieure de chacune des première et deuxième sous-parties.

7. Matériau selon la revendication 4, comprenant en outre une troisième sous-partie (C) formée à partir d'un troisième matériau généralement plan, la troisième sous-partie étant couplée, sans chevauchement, le long d'une face de bord à une face de bord d'au moins une parmi la première sous-partie et la deuxième sous-partie, dans lequel le troisième matériau diffère de chacun des premier et deuxième matériaux par au moins une propriété physique.

8. Matériau selon la revendication 4, dans lequel ladite au moins une propriété physique de laquelle diffèrent les premier et deuxième matériaux est choisie dans le groupe constitué d'élasticité, masse volumique, rigidité et perméabilité à l'air.

9. Ensemble casque comprenant :
une première sous-partie formée à partir d'un premier matériau généralement plan ; et
une deuxième sous-partie formée à partir d'un deuxième matériau généralement plan, dans lequel le deuxième matériau diffère du premier matériau par au moins une propriété physique,
**caractérisé en ce que** la deuxième sous-partie est couplée le long d'une face de bord de celle-ci à une face de bord de la première sous-partie sans chevaucher la première sous-partie.

10. Ensemble casque selon la revendication 9, comprenant en outre une troisième sous-partie formée à partir d'un troisième matériau généralement plan, la troisième sous-partie étant couplée, sans chevauchement, le long d'une face de bord de celle-ci à une face de bord d'au moins une parmi la première sous-partie et la deuxième sous-partie, dans lequel le troisième matériau diffère de chacun des premier et deuxième matériaux par au moins une propriété physique.

11. Ensemble casque (30) selon la revendication 10, comprenant en outre une quatrième sous-partie formée à partir d'un quatrième matériau généralement plan qui diffère de chacun des premier, deuxième et troisième matériaux par au moins une propriété physique, la quatrième sous-partie étant couplée, sans chevauchement, le long d'une face de bord de celle-ci à une face de bord d'au moins une parmi la première sous-partie, la deuxième sous-partie ou la troisième sous-partie, dans lequel :
la première sous-partie (A₁) comprend un panneau arrière (32) conçu pour être placé sur une partie arrière inférieure de la tête d'un patient lorsque l'ensemble casque est placé sur la tête d'un patient ;
la quatrième sous-partie (D₁) comprend une sangle supérieure (34) conçue pour être placée au niveau ou autour du sommet de la tête d'un patient lorsque l'ensemble casque est placé sur la tête d'un patient ;
la deuxième sous-partie (B₁) comprend une région disposée adjacente, et couplée à la sangle supérieure, la deuxième sous-partie étant conçue pour fixer la sangle supérieure en place sur la tête d'un patient lorsque l'ensemble casque est placé sur la tête d'un patient ;
la troisième sous-partie (C₁) comprend un élément courbé (35) conçu pour être placé, et maintenir un angle prévu, autour de l'oreille d'un patient lorsque l'ensemble casque est placé sur la tête d'un patient ;
le premier matériau est fortement élastique, possédant une première élasticité ;
le deuxième matériau possède une deuxième élasticité inférieure à la première élasticité ;
le troisième matériau possède une troisième élasticité inférieure à la deuxième élasticité ; et
le quatrième matériau possède une quatrième élasticité inférieure à la première élasticité et à la deuxième élasticité.

12. Ensemble casque (40) selon la revendication 10, dans lequel :
la première sous-partie (A₂) comprend une partie généralement en forme de c (41) qui est conçue pour être placée généralement autour de l'oreille d'un patient pour fournir de la solidité et empêcher l'ensemble casque de se détendre ou de s'affaisser autour de l'oreille du patient lorsque l'ensemble casque est placé sur la tête d'un patient ;
la troisième sous-partie (C₂[SP1]) comprend un certain nombre de sangles arrière (44, 46) conçues pour être placées à l'arrière de la tête d'un patient lorsque l'ensemble casque est placé sur la tête d'un patient ;
la deuxième sous-partie (B₂) comprend :
une partie d'un élément de sangle avant (42) conçue pour être placée sous l'oeil d'un patient et est conçue pour empêcher l'élément de sangle avant d'empiéter sur l'oeil du patient lorsque l'ensemble casque est placé sur la tête d'un patient, et
une autre partie (43) disposée adjacente, et couplée, à la partie en forme de x, l'autre partie étant conçue pour stabiliser le nombre de sangles arrière lorsque l'ensemble casque est placé sur la tête d'un patient ;
le premier matériau est un matériau souple, généralement non élastique ;
le deuxième matériau est moins souple que le premier matériau ; et
le troisième matériau est plus élastique que le premier matériau.

13. Ensemble casque (50) selon la revendication 10, dans lequel :
la troisième sous-partie (C₃) comprend :
une sangle supérieure (57) conçue pour être placée au niveau ou autour du sommet de la tête d'un patient lorsque l'ensemble casque est placé sur la tête d'un patient, et
une paire de sangles avant (58, 59) conçues pour être placées généralement le long de la joue d'un patient lorsque l'ensemble casque est placé sur la tête d'un patient ;
la deuxième sous-partie (B₃) comprend :
un élément de raccordement (55) couplé entre la paire de sangles avant, et
une partie de stabilisation disposée autour d'une base de la sangle supérieure ;
la première sous-partie (A3) comprend une pluralité de sangles arrière (52, 54, 56) conçues pour être placée le long de l'arrière de la tête d'un patient lorsque l'ensemble casque est placé sur la tête d'un patient ;
le premier matériau possède une première élasticité ;
le deuxième matériau possède une deuxième élasticité inférieure à la première élasticité ; et
le troisième matériau possède une troisième élasticité inférieure à la première élasticité et supérieure à la deuxième élasticité.

14. Ensemble casque (60) selon la revendication 10, dans lequel :
la première sous-partie (A₄) comprend un certain nombre de parties conçues pour être placées autour des oreilles d'un patient lorsque l'ensemble casque est placé sur la tête d'un patient, le nombre de parties étant conçu pour limiter l'allongement global de l'ensemble casque ;
la troisième sous-partie comprend une sangle supérieure (66) conçue pour être placée au niveau ou autour du sommet de la tête d'un patient lorsque l'ensemble casque est placé sur la tête d'un patient ;
la deuxième sous-partie (B₄) comprend :
une première partie (65) conçue pour être placée généralement au-dessus de l'oreille d'un patient lorsque l'ensemble casque est placé sur la tête d'un patient, la première partie étant couplée entre des parties de la première sous-partie et à la sangle supérieure, la première partie étant conçue pour réduire la mobilité de la sangle supérieure, et
une deuxième partie (67) conçue pour être placée à l'avant de l'oreille d'un patient lorsque l'ensemble casque est placé sur la tête d'un patient, la deuxième partie étant couplée à des parties de la première sous-partie ;
le premier matériau possède une première élasticité ;
le deuxième matériau possède une deuxième élasticité inférieure à la première élasticité ; et
le troisième matériau possède une troisième élasticité supérieure à la première élasticité et à la deuxième élasticité.

15. Ensemble casque (70) selon la revendication 10, dans lequel :
l'ensemble casque comprend un panneau arrière (72) conçu pour être placé sur une partie arrière de la tête d'un patient lorsque l'ensemble casque est placé sur la tête d'un patient ;
la première sous-partie (A₅) comprend une partie centrale (74) du panneau arrière ;
la deuxième sous-partie (B₅) comprend une paire de parties secondaires (76) disposées de chaque côté de la partie centrale ;
la troisième sous-partie (C₅) comprend un certain nombre de sangles (78) couplées aux parties secondaires ;
le premier matériau possède une première élasticité ;
le deuxième matériau est un matériau généralement rigide possédant une deuxième élasticité inférieure à la première élasticité ; et
le troisième matériau possède une troisième élasticité inférieure à la première élasticité et supérieure à la deuxième élasticité.
